Europäisches Patentamt

European Patent Office

Office européen des brevets

(19)

(11) Veröffentlichungsnummer: **0 096 311**
**B1**

(12) **EUROPÄISCHE PATENTSCHRIFT**

(45) Veröffentlichungstag der Patentschrift: **17.05.89**

(51) Int. Cl.⁴: **G 01 N 15/08**

(21) Anmeldenummer: **83105256.8**

(22) Anmeldetag: **27.05.83**

(54) **Prüfeinrichtung zur Messung der Luftdurchlässigkeit von textilen Materialbahnen.**

(30) Priorität: **08.06.82 DE 8216624 u**
**25.02.83 DE 8305292 u**

(43) Veröffentlichungstag der Anmeldung:
**21.12.83 Patentblatt 83/51**

(45) Bekanntmachung des Hinweises auf die
Patenterteilung:
**17.05.89 Patentblatt 89/20**

(84) Benannte Vertragsstaaten:
**AT BE CH DE FR GB IT LI NL SE**

(56) Entgegenhaltungen:
**GB-A-2 001 444**
**US-A-3 604 246**
**US-A-4 311 037**

(73) Patentinhaber: **Thomas Josef Heimbach GmbH
& Co.
An Gut Nazareth 73
D-5160 Düren-Mariaweiler (DE)**

(72) Erfinder: **Pulsmeier, Harald
Hamischerstrasse
D-5163 Langerwehe-Heistern (DE)**
Erfinder: **Best, Walter, Dr. Dipl.-Chem.
Merowingerstrasse 8
D-5160 Düren (DE)**

(74) Vertreter: **Paul, Dieter-Alfred, Dipl.-Ing.
Fichtestrasse 18
D-4040 Neuss 1 (DE)**

Courier Press, Leamington Spa, England.

## Beschreibung

Die Erfindung betrifft eine Prüfeinrichtung zur Messung der Luftdurchlässigkeit von textilen Materialbahnen, insbesondere von Papiermaschinenfilzen und -sieben, mit einer Meßgabel mit im wesentlichen parallel und im Abstand zueinander verlaufenden Schenkeln, an deren freien Enden je ein Rohrstutzen angebracht ist, von denen einer mittels einer Einspanneinrichtung zum Einspannen der Materialbahn zwischen den Rohrstutzen gegen den anderen bewegbar ist und von denen einer über einen Luftkanal, insbesondere Saugkanal, mit einem durch einen Motor angetriebenen Lüfter bzw. Pumpe verbunden ist, wobei in dem Luftkanal zumindest eine Steuereinrichtung zur Einstellung des Volumenstroms, zumindest ein Unterdruckmesser—vorzugsweise im Bereich des angeschlossenen Rohrstutzens—sowie zumindest ein Luftdurchfluß oder Luftvolumenmesser angeordnet sind.

Papiermaschinenfilze und -siebe werden einer Luftdurchlässigkeitsprüfung unterzogen, da sie einen Parameter für die Eigenschaften dieser textilen Materialbahnen bilden. Hierzu werden Prüfeinrichtungen verwendet, die eine Meßgabel mit parallel zueinander verlaufenden Schenkeln aufweisen, zwischen denen die Materialbahnen mittels einer handbetätigten Einspanneinrichtung eingeklemmt werden können. Die Einspannung erfolgt zwischen zwei Rohrstutzen, von denen der eine fest an einem Schenkel und der andere an einem durch die Einspanneinrichtung bewegbaren Teil des anderen Schenkels angeordnet sind. Nach der Einspannung liegen die Rohrstutzen im wesentlichen koaxial zueinander.

An dem festen Rohrstutzen ist ein Unterdruckabnehmer sowie ein langer, flexibler Saugschlauch angeschlossen. Letzterer führ zu einer Rotametereinheit, die der Durchflußmessung dient. Von der Rotametereinheit setzt sich der Saugschlauch fort zu einem von einem Elektromotor angetriebenen Sauglüfter. Zur Einstellung des Meßdifferenzdruckes dienen von Hand zu betätigende Ventile oberhalb der Rotametereinheit. Durch Probieren muß dabei der jeweilige Meßbereich ermittelt werden. Hierzu muß einzeln jedes Ventil geöffnet werden, bis ein für den Meßbereich geeignetes Rotameter gefunden ist.

Mit einer derartigen Prüfeinrichtung werden Papiermaschinenfilze und -siebe unter Anwendung der DIN 53 887 gemessen. Allerdings hat sich diese Messung mit solchen Prüfeinrichtungen in vielfacher Hinsicht als ungenügend erwiesen.

So sind die Meßwerte, die mit verschiedenen Probenquerschnitten erzielt werden, nicht vergleichbar. Auch liegen die Meßwerte für gleiche Meßproben bei den einzelnen Meßgeräten oft weit auseinander. Eine Umrechnung der einzelnen Werte, um diese miteinander vergleichen zu können, war bisher nicht möglich. Die Angaben verschiedener Hersteller gaben somit keinen Aufschluß über die Luftdurchlässigkeit in Vergleich stehender Filze oder Siebe.

Dies hängt nicht nur mit der Meßmethode zusammen, sondern auch mit der Gestaltung der einzelnen Prüfeinrichtung selbst. Ihre strömungstechnische Ausbildung ist bisher eher mangelhaft. Entsprechend ist die Saugströmung oft gestört und unstetig. In den langen und flexiblen Saugschläuchen entstehen hohe Druckverluste. Außerdem ist der Anpressdruck der Rohrstutzen am Filz oder Sieb von Hand kaum reproduzierbar einzustellen. Eine Minimierung der seitlich einströmenden Fehlluft ist deshalb fast unmöglich.

Weiterer Schwachpunkt der bisherigen Prüfrichtungen sind die Rotameter zur Ermittlung der durch die Probe gesaugten Luftmenge. Da ihr Meßbereich in der Regel nur sehr gering ist, müssen mehrere Meßgeräte vorgesehen werden. Selbst dies reicht aber für offene Siebe nicht aus. Zudem neigen die Schwebekörper in den Rotametern zum Springen und Pumpen, die das Ablesen des Meßwertes außerordentlich schwierig gestaltet. Dies könnte nur durch wesentliches Herabsetzen der Druckverluste vermieden werden. Berechnungen haben jedoch gezeigt, daß eine solche Herabsetzung nicht erreichbar ist.

Des weiteren wird bei den bekannten Prüfrichtungen der Unterdruck nur zu Beginn der Messung vorgegeben und dann nicht mehr nachgeregelt. Druckschwankungen schlagen somit voll auf das Meßergebnis durch. Schließlich ist die Handhabung der bekannten Prüfeinrichtungen außerordentlich mühsam, da zum Einspannen der Meßgaben eine Person häufig auf dem Boden arbeiten muß und eine weitere Person für den Einstell- und Meßprozeß erforderlich ist.

Der Erfindung liegt die Aufgabe zugrunde, die Prüfeinrichtung der eingangs genannten Art so zu verbessern, daß mit ihr reproduzierbare und nach Möglichkeit auch in weiten Bereichen umrechenbare und zudem genaue Meßwerte erreichbar sind, wobei sich die Prüfeinrichtung durch eine einfache Handhabung auszeichnen soll.

Diese Aufgabe wird erfindungsgemäß dadurch gelöst, daß die Meßgabel starre, von einem Rahmen, insbesondere einem Gehäuseteil, für den Lüfter und dessen Motor ausgehende Schenkel aufweist, wobei einer der Rohrstutzen im wesentlichen koaxial zu dem anderen Rohrstutzen relativ dem betreffenden Schenkel verschiebbar ist und der andere Rohrstutzen mit dem als Luftrohr ausgebildeten Luftkanal verbunden ist, das in dem betreffenden Schenkel zum großen Teil gerade verläuft.

Vorteilhaft bei dieser erfindungsgemäßen Prüfeinrichtung ist, daß die Prüfeinrichtung nunmehr ein einheitliches Gerät mit in diesem untergebrachtem Lüfter bzw. Pumpe ist, wobei die Führung des Luftrohrs in einem der Schenkel dazu ausgenutzt wird, eine relativ lange Beruhigungsstrecke zu erreichen, die exaktere Messungen ermöglicht. Hinzu kommt, daß durch den im wesentlichen koaxial beweglichen Rohrstutzen gesichert ist, daß beide Rohrstutzen nach Einklemmung der jeweiligen Materialbahnen genau übereinanderliegen, was zur Erreichung einer möglichst exakt begrenzten Prüffläche wichtig ist.

Auch läßt sich eine solche Prüfeinrichtung leichter handhaben, da sie nicht mehr aus verschiedenen Teilen, sondern eine einheitliche Vorrichtung bildet.

In Ausbildung der Erfindung ist vorgesehen, daß der mit dem Luftrohr verbundene Rohrstutzen als Doppelrohrstutzen mit jeweils koaxial zueinander angeordneten Innen und Außenrohrteilen ausgebildet ist, daß das Luftrohr an das Innenrohrteil des betreffenden Doppelrohrstutzens angeschlossen und das Außenrohrteil an ein in dem betreffenden Schenkel verlaufendes, zwischen Luftdurchlaß- bzw. Luftvolumenmesser und Sauglüfter in das Luftrohr einmündendes Parallelrohr verbunden ist, in dem ein Steuerventil zur Einstellung des Unterdrucks angeordnet ist, und daß Außenrohrteil und Innenrohrteil des unbewegbaren Doppelrohrstutzens an einem zweiten Differenzdruckmesser angeschlossen sind.

Über diesen zweiten Differenzdruckmesser kann dann das Steuerventil in dem Parallelrohr so eingestellt werden, daß die Drücke im Innenraum des Innenrohrteils und im Ringraum zwischen Innen- und Außenrohrteil gleich sind, also keine Druckdifferenz herrscht. Auf diese Weise ist es ausgeschlossen, daß Falschluft in das Innenrohrteil und damit zu den Luftdurchfluß- oder Luftvolumenmessern gelangt, wodurch eine Meßwertverfälschung eintreten würde. Gemessen wird also die tatsächlich über den Querschnitt des Innenrohrteils durch die Materialbahn hindurchgehende Luft.

Vorzugsweise sind beide Rohrstutzen als Doppelrohrstutzen ausgebildet, wobei die gegenüberliegenden Ränder von Innen- und Außenrohrteilen jeweils deckungsgleich sind. Dabei sollte der freie Abstand zwischen Innen-und Außenrohrteil(en) mindestens 5 mm betragen.

Das bzw. die Außenrohrteil(e) sind zweckmäßigerweise jeweils über Stege starr mit dem bzw. den Innenrohrteil(en) verbunden.

Von besonderem Vorteil ist es, wenn das Steuerventil in dem Parallelrohr mit einem Steuermotor verbunden wird, wobei der Steuermotor an einem mit dem zweiten Differenzdruckmesser in Verbindung stehenden Unterdruckregler angeschlossen ist. Auf diese Weise wird automatisch eine evtl. auftretende Druckdifferenz zwischen Innenrohrteilen und Außenrohrteilen durch entsprechende Nachstellung des Steuerventils ausgeglichen. Eingriff und Kontrolle von außen sind dann nicht mehr erforderlich.

Es ist des weiteren vorgesehen, daß der bewegbare Rohrstutzen strömungsgünstig gerundete Lufteintrittsöffnung(en) aufweist. Dies war bei den bisher bekannten Prüfeinrichtungen nicht der Fall und sorgte dafür, daß insbesondere bei hohen Luftgeschwindigkeiten, wie sie bei der Prüfung von offenen Sieben auftreten, Saugstrahlkontraktionen auftraten, die den wirksamen Prüfquerschnitt verringerten und so zu nicht vergleichbaren Ergebnissen führten. Dies wird durch die gerundete Lufteintrittsöffnung(en) vermieden. Zusätzlich sollte der bewegbare Rohrstutzen möglichst frei von strömungshemmenden und querschnittsverändernden Elementen sein. Des weiteren sollten beide Rohrstutzen und auch das Luftrohr eine polierte Innenfläche aufweisen. Durch diese Maßnahmen entstehen geringste Einlauf- und Durchströmverluste und eine optimale Beaufschlagung der Probenfläche.

Es ist ferner vorgesehen, daß der bewegbare Rohrstutzen über eine Parallelogrammführung verschiebbar ist. Noch besser ist es, eine Linearführung vorzusehen, die zweckmäßigerweise Linearführungselemente aufweist, die jeweils aus einer Führungsstange und einer diese umfassende Führungsnut bestehen.

Gemäß einem weiteren Merkmal ist vorgesehen, daß für die Bewegung des einen Rohrstutzens ein Motor zur Erzeugung eines definierten Anpressdrucks vorgesehen ist. Hierzu eignet sich insbesondere ein als doppelt wirkender Hydraulik- oder Pneumatikzylinder ausgebildeter Motor. Dies ermöglicht eine stufenlose Einstellung der Einspannkraft und gewährleistet des weiteren, daß die einmal eingestellte Einspannkraft konstant bleibt, so daß die Proben immer der gleichen Flächenpressung unterliegen. Zweckmäßigerweise sollte eine analoge oder digitale Anzeige für den Einspanndruck des bewegbaren Doppelrohrstutzens vorgesehen sein. Die Verbindung des bewegbaren Doppelrohrstutzens mit dem Hydraulik- oder Pneumatikzylinder kann über einen Kniehebel geschehen.

In weiterer Ausbildung ist vorgesehen, daß die Rohrstutzen als Ganzes auswechselbar sind. Dies kann beispielsweise durch Einschraubung in Passende Fassungen oder durch einen Bajonettverschluß geschehen. Auf diese Weise können Rohrstutzen mit verschiedenen freien Querschnittsflächen verwendet werden.

Es ist ferner vorteilhaft, wenn in alle einander zugewandten Stirnflächen der Rohrstutzen jeweils zumindest ein, vorzugsweise mehrere, konzentrische Rundschnurdichtringe mit quadratischem oder rundem Querschnitt eingesetzt sind. Auf diese Weise wird eine Labyrinth-Wirkung erzielt, die das seitliche Einfließen von Fehlluft, insbesondere bei hohen Luftgeschwindigkeiten, vermeiden hilft.

Zum Zwecke der gleichmäßigen Beaufschlagung der Probenfläche und einer möglichst strömungsgünstigen Führung ist es des weiteren zweckmäßig, wenn das Luftrohr um einen Betrag, der zumindest das Zweifache seines Durchmessers beträgt, koaxial zum mit ihm verbundenen Innenrohrteil des Doppelrohrstutzens verläuft.

Es ist ferner vorgeschlagen, daß der Luftdurchfluß- bzw. Luftvolumenmesser im sauglüfterseitigen Endbereich des geraden Teils des Saugrohrs angeordnet ist. Diese Anordnung bietet eine genaue Messung des Luftdurchflusses bzw. Luftvolumens, da das lange gerade Rohr dann als Beruhigungsstrecke wirkt.

Als besonders geeignet für die Luftdurchflußmessung haben sich Meßgeräte auf thermischer Basis erwiesen. Hier kommt insbesondere das Hitzdrahtanemometer infrage, noch besser jedoch ein sogenannter Heißfilmsensor, da er unempfindlicher gegenüber mitgerissenen Staubteilchen und Verunreinigungen ist. Diese Meßgeräte zeichnen sich durch einen sehr hohen Meßbereich von 1: 100 und eine sehr große Genauigkeit aus. Der gesamte Meßbereich der Prüfeinrichtung kann auf diese Weise mit einem einzigen Meßaufnehmer abgedeckt werden. Alternativ dazu kann vorgesehen sein, daß der Luftdurchflußmesser als dritter Differenzdruckmesser, insbesondere zusammen mit einer Düse oder Drossel im Luftkanal, ausgebildet ist. Selbstverständlich bietet es sich auch hier an, daß der Luftdurchflußmesser mit einer analogen oder digitalen Meßwertanzeige verbunden ist.

Weiterhin ist vorgesehen, daß der erste Differenzdruckmesser je eine Druckentnahme in den beiden Rohrstutzen bzw. deren Innenrohrteil(en) aufweist. Bisher wurde immer nur der Unterdruck in der Saugleitung abgenommen. Dabei ist es besonders vorteilhaft, wenn die Rohrstutzen bzw. deren Innenrohrteil(en) über den Umfang verteilt mehrere — zumindest drei — Druckentnahmen aufweisen, die über je einen Ringkanal ggf. in dem bzw. den Innenrohrteil(en) verbunden sind, da auf diese Weise eine Mittelung erreicht wird. Aus gleichem Grund sollte auch das Außenrohrteil des festen Doppelrohrstutzens einen Ringkanal mit mehreren über den Umfang verteilten Druckentnahmen angeordnet sein.

Es ist ferner vorgeschlagen, daß an das Saugrohr zwischen Luftdurchfluß- bzw. Luftvolumenmesser und Sauglüfter zumindest eine Bypass-Leitung mit darin angeordnetem Regelventil angeschlossen ist. Sofern mit einer Bypass-Leitung nicht der gesamte Regelbereich abgedeckt werden kann, kann es zweckmäßig sein, eine größere Bypass-Leitung mit einem Grobregelventil und eine kleinere Bypass-Leitung mit einem Feinregelventil vorzusehen. Diese Aufteilung ermöglicht es, einerseits den vom Sauglüfter erzeugten Unterdruck fast vollständig zu vernichten, um gewährleisten zu können, daß die Regelung auch bei sehr dichten Proben noch funktionsfähig ist. Andererseits ist—nach ungefährer Anpassung durch das Grobregelventil in der größeren Bypass-Leitung—eine sehr präzise Regelung durch das Feinregelventil in der kleineren Bypass-Leitung erzielbar, wodurch eine verbesserte Genauigkeit der Meßwerte erreicht wird. Dabei kann das Grob- und Feinregelventil jeweils mit einem Verstellmotor versehen sein, wobei der Verstellmotor des Grobregelventils an einem Dreipunktregler mit einstellbarem Totbereich angeschlossen wird. Innerhalb dieses Totbereichs übernimmt das Feinregelventil die Konstanthaltung des Unterdrucks. Hierzu ist es vorgesehen, daß Differenzdrucknehmer und Regelventil(e) in einem Regelkreis zur Einstellung und zum Konstanthalten des Differenzdrucks angeordnet sind. Auf diese Weise können evtl. Druckänderungen nicht mehr in der bisherigen Form in die Meßergebnisse durchschlagen.

Es wird des weiteren vorgeschlagen daß die Meßgabel mit allen Teilen in einem Gehäuse untergebracht ist, so daß das Gerät einheitlich gehandhabt werden kann und demgemäß auch nur eine Bedienungsperson erfordert. Sie kann beispielsweise ein Rollenfahrwerk aufweisen, das es ermöglicht, die Meßgabel in die Probe einzufahren. Es kann des weiteren auch eine Linearführungseinrichtung vorgesehen sein, in der die Meßgabel verschieblich gelagert ist und die zweckmäßigerweise auf einem Fahrgestell gelagert ist.

Um breite Papiermaschinenfilze und -siebe auch im mittleren Bereich durchmessen zu können, sollte die Meßgabel zumindest eine 1,5 m lange Öffnung haben, wobei Werte um 2 m erwünscht sind.

Schließlich wird vorgeschlagen, daß der Lüfter als Sauglüfter ausgebildet ist, der bei offener Prüfeinrichtung einen Unterdruck von zumindest 2 mbar erzeugt. Dies ermöglicht ein Meßverfahren, bei dem der Verlust der Prüfeinrichtung eliminiert wird. Hierzu wird zunächst der Luftdurchsatz bei offener Prüfeinrichtung bei definiertem Differenzdruck gemessen. Anschließend wird die Probe eingespannt und der Luftdurchsatz bei demselben Differenzdruck gemessen. Durch Subtraktion der beiden Meßwerte erhält man den Luftdurchsatz, der der Probe zuzuordnen ist. Auf diese Weise werden umrechenbare Werte für die Luftdurchlässigkeit ermittelt.

In der Zeichnung ist die Erfindung an Hand eines schematisch dargestellten Ausführungsbeispiels näher veranschaulicht. Es zeigen:

Figur 1 eine Prüfeinrichtung für Papiermaschinenfilze und -siebe in der Seitenansicht;

Figur 2 die Innenteile der Prüfeinrichtung gemäß Figur 1 in der Seitenansicht;

Figur 3 eine Schnittdarstellung entlang der Linie A—B in Figur 2;

Figur 4 eine vergrößerte Schnittdarstellung durch die Doppelrohrstutzen der Prüfeinrichtung gemäß den Fig. 1 bis 3.

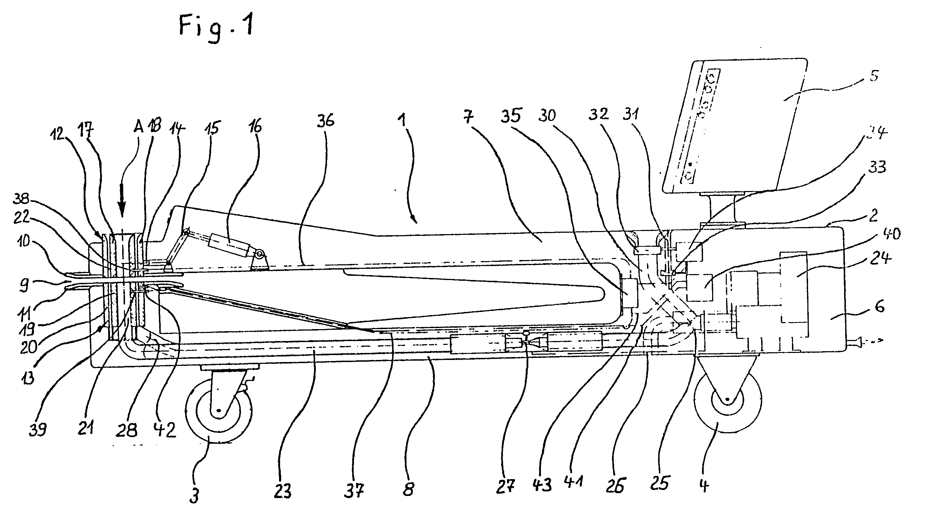

Die in den Figuren 1 bis 3 zumindest teilweise dargestellte Prüfeinrichtung 1 ist für Papiermaschinenfilze und -siebe vorgesehen. Sie besteht im wesentlichen aus einer Meßgabel 2, darunter angeordneten Rollen 3, 4 sowie einem aufgesetzten, um 270° drehbar gelagerten Anzeige- und Bedienungspult 5, in dem sich die gesamte Elektronik der Prüfeinrichtung 1 befindet. Die Rollen 3, 4 dienen dazu, daß die Prüfeinrichtung 1 in gewünschter Weise verfahren werden kann.

Die Meßgabel 2 hat von einem Gehäuseteil 6 ausgehende, hohl und deshalb selbsttragende Schenkel 7, 8 gleicher Länge, die übereinander angeordnet sind. An den freien Enden lassen sie einen Meßspalt 9 frei, der durch zwei gegenüberliegende Führungsteller 10, 11 begrenzt wird. Zwischen diese Führungsteller 10, 11 kann eine textile Materialbahn, beispielsweise ein Papiermaschinenfilz, eingeführt werden, wobei die grosse Maulbreite der Meßgabel 2 ein Einfahren mit entsprechendem Abstand zum Rand ermöglicht.

Koaxial zu den Führungstellern 10, 11 sind in den Schenkeln 7, 8 Doppelrohrstutzen 12, 13 deckungsgleich angeordnet. Der obere Doppelrohrstutzen 12 ist in dem oberen Schenkel 7 verschieblich geführt, und zwar mittels einer hier nicht näher dargestellten Präzisionslinearführung in exakt axialer Richtung. Diese Linearführung besteht aus drei am Umfang des Doppelrohrstutzens 12 verteilt angeordneten, in axialer Richtung verlaufenden Führungsstangen, die in mit dem Schenkel 7 verbundenen Führungsnuten laufen. Bei der Bewegung bleibt der Doppelrohrstutzen 12 somit immer deckungsgleich mit dem unteren Doppelrohrstutzen 13.

Der obere Doppelrohrstutzen 12 ist gelenkig mit einer Betätigungsgabel 14 verbunden, die fest an einem Kniehebel 15 angebracht ist. Letzterer ist im Schenkel 7 ortsfest gelagert und am vom Doppelrohrstutzen 12 entfernten Ende gelenkig mit einem doppelt wirkenden Pneumatikzylinder 16 verbunden. Dieser wird über ein durch Tastendruck betätigtes Elektroventil gesteuert, was hier nicht näher dargestellt ist. Der Druck und damit die Einspannkraft kann stufenlos eingestellt und dann konstant gehalten werden. Durch eine Anzeige ist er auch jederzeit reproduzierbar. Die Entspannung erfolgt ebenfalls über Tastendruck.

Der obere Doppelrohrstutzen 12 besteht aus einem Innenrohrteil 17 und einem dieses mit Abstand umgebenden, koaxial angeordneten Außenrohrteil 18. Die oberen Enden von Innenrohrteil 17 und Außenrohrteil 18 sind strömungsgünstig ausgeformt, so daß in diesem Bereich keine Verwirbelungen auftreten und die Probenfläche optimal über ihre gesamte Fläche beaufschlagt wird. Der Pfeil A gibt dabei die Strömungsrichtung der Luft an. Zusätzlich sind Innen- und Außenrohrteil 17, 18 innenseitig poliert.

Der untere Doppelrohrstutzen 13 ist ortsfest in dem unteren Schenkel 8 eingebaut. Auch er besteht aus einem Innenrohrteil 19 und einem Außenrohrteil 20, wobei die Innenrohrteile 17, 19 und die Außenrohrteile 18, 20 der beiden Doppelrohrstutzen 12, 13 jeweils deckungsgleich liegen. Auf diese Weise ergeben sich ein Innenluftkanal 21, der von den Innenrohrteilen 17, 19 umschlossen ist, sowie ein im Querschnitt ringförmiger Außenluftkanal 22, der zwischen den Außenwänden der Innenrohrteile 17, 19 und den Innenwänden der Außenrohrteile 18, 20 verläuft.

An dem unteren Ende dieses Innenrohrteils 19 ist ein Saugrohr 23 angeschlossen, das zunächst gerade nach unten und nach einer Biegung waagerecht durch den ganzen Schenkel 8 verläuft. Auf diese Weise ergibt sich eine lange Beruhigungsstrecke für die Rohrströmung. Das Saugrohr 23 mündet in einen im Gehäuseteil 6 untergebrachten Sauglüfter 24. Von diesem geht eine Abluftleitung 25 mit einer nach unten gerichteten Mündung 26 ab. Zur Verringerung der Strömungsgeräusche ist zusätzlich ein hier nicht sichtbarer Schalldämpfer eingebaut.

Im sauglüfterseitigen Bereich des Saugrohrs 23 ist ein nach dem thermischen Meßprinzip arbei-tender Heißfilmsensor 27 als Luftdurchflußmes-ser angeordnet. Durch die in diesem Bereich beruhigte Luftströmung arbeitet er sehr genau.

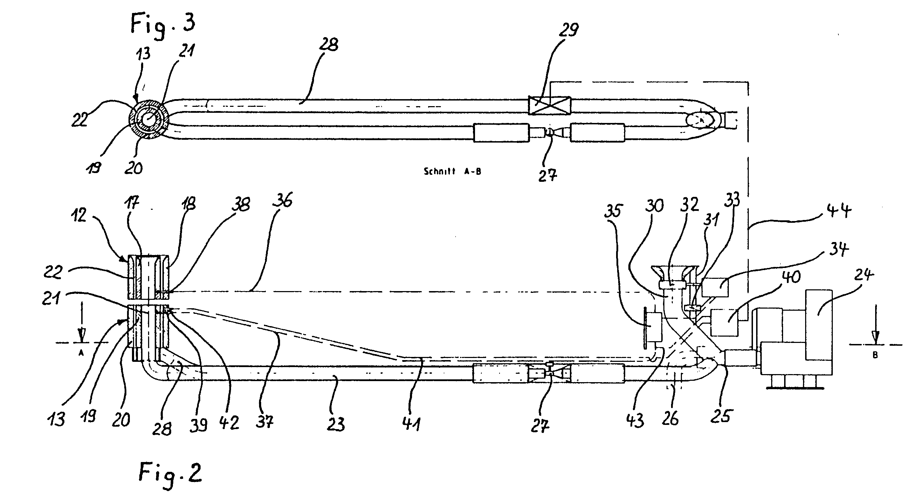

Der Außenluftkanal 22 ist am unteren Ende des Außenrohrteils 20 an ein Parallelrohr 28 angeschlossen, das, wie insbesondere Figur 3 zeigt, parallel neben dem Saugrohr 23 verläuft und in dieses kurz vor dem Sauglüfter 24, also hinter dem Heißfilmsensor 27, mündet. In diesem Paral-lelrohr 28 ist ein Steuerventil 29 eingebaut, mit dem der Unterdruck im Parallelrohr 28 eingestellt werden kann.

An der Einmündung des Parallelrohrs 28 in das Saugrohr 23 zweigt eine erste Bypass-Leitung 30 ab. Von dieser wiederum geht eine zweite Bypass-Leitung 31 geringeren Querschnitts aus. Die erste Bypass-Leitung 30 hat ein Grobregelventil 32 und die zweite ein Feinregelventil 33, die beide mit einer Steuereinrichtung 34 verbunden sind. Die Steuereinrichtung 34 beinhaltet eine Regelelektronik sowie Stellmotoren für die Regelventile 32, 33. Sie ist mit einem Differenzdruckaufnehmer 35 gekuppelt, der über Leitungen 36, 37 mit Druckabnehmern 38, 39 in den Innenrohrteilen 17, 19 der Doppelrohrstutzen 12, 13 verbunden ist.

Im Bereich der Bypass-Leitungen 30, 31 ist eine Regeleinrichtung 40 angeordnet, die über eine Leitung 41 mit einem Druckabnehmer 42 im Außenrohrteil 20 verbunden ist. Des weiteren ist diese Regeleinrichtung 40 über eine Leitung 43 an die Leitung 37 angeschlossen. Die Regeleinrichtung 40 erhält somit die Drücke im Innenluftkanal 21 und im Außenluftkanal 22 im Doppelrohrstutzen 13. Von der Regeleinrichtung 40 geht eine Ausgangsleitung 44 ab, die zu einem Stellmotor für das Steuerventil 29 geht.

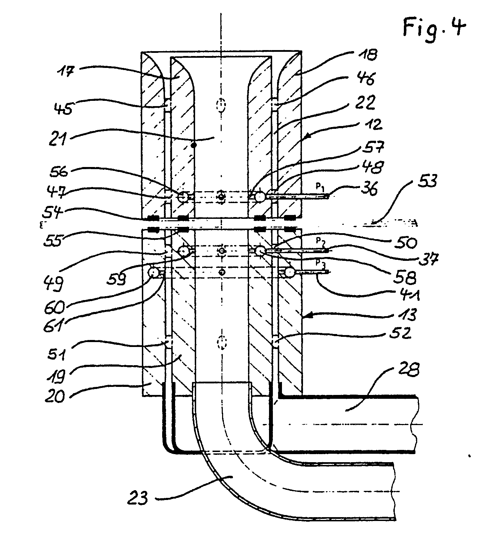

Figur 4 zeigt eine vergrößerte Darstellung der Doppelrohrstutzen 12, 13 im Vertikalschnitt. Die beiden Innenrohrteile 17, 19 sind über Tragrippen 45, 46, 47, 48, 49, 50, 51, 52 an den Außenrohrteilen 18, 20 befestigt, und zwar so, daß sich ein gleichmäßiger Abstand und damit ein ringförmiger Außenluftkanal 22 bildet. Zwischen den auf-einander zugerichteten Stirnseiten der Doppel-rohrstutzen 12, 13 ist ein Sieb 53 eingespannt. In die Stirnseiten sind hierfür im Querschnitt rechteckige Rundschnurringe 54, 55 eingelassen, die das Eintreten von Falschluft verhindern sollen. An den Innenluftkanal 21 schließt sich das Saugrohr 23 an, während das parallele Rohr 28 den Außenluftkanal 22 fortsetzt.

In das Innenrohrteil 17 des oberen Doppelrohrstutzens 12 ist ein Ringkanal 56 eingelassen, der über vier über den Umfang verteilte Bohrungen 57 mit dem Innenluftkanal 21 verbunden ist. Von diesem Ringkanal 56 geht die Leitung 36 ab, wobei sie durch die Tragrippe 48 hindurchgeführt wird. Im Innenrohrteil 19 des unteren Doppelrohrstutzens 13 ist ebenfalls ein Ringkanal 58 vorgesehen, von dem die Leitung 37 abgeht und der über gleichfalls vier Bohrungen 59 mit dem Innenluftkanal 21 verbunden ist.

Das Außenrohrteil 20 des unteren Doppelrohrstutzens 13 weist einen weiteren Ringkanal 60 auf, der über vier Bohrungen 61 Verbindung mit dem Außenluftkanal 22 hat und an den die Leitung 41 zu der Regeleinrichtung 40 angeschlossen ist.

Der Meßvorgang mit der dargestellten Prüfeinrichtung 1 gestaltet sich wie folgt:

Nachdem das Sieb 53 zwischen den Doppelrohrstutzen 12, 13 durch Betätigung des Pneumatikzylinders 16 mit einem definiertem Druck eingespannt worden ist, wird bei eingeschaltetem Sauglüfter 24 der Steuereinrichtung 34 eine gewünschte Druckdifferenz an der Probe vorgegeben, beispielsweise 2 mbar. Es tritt dann ein Dreipunktregler für die Steuerung des Grobregelventils 32 in Aktion, der einen eingestellten Totbereich hat, beispielsweise von 1,8 bis 2,2 mbar. Das Grobregelventil 32 wird nun aus der Offenstellung so weit zugefahren, bis dem Dreipunktregler ein Differenzdruck von 1,8 mbar gemeldet wird. Dann übernimmt die weitere Regelung das Feinregelventil 33, mit dem der Differenzdruck auf 2,0 mbar hochgefahren und dann mit größtmöglicher Genauigkeit eingehalten wird.

Gleichzeitig wird der Stellmotor des Steuerventils 29 von der Regeleinrichtung 40 immer so eingestellt, daß im Innenluftkanal 21 und in dem daran anschließenden Saugrohr 23 sowie in dem Außenluftkanal 22 und dem anschließenden Parallelrohr 28 immer der gleiche Druck herrscht. Auf diese Weise wird vermieden, daß in den Innenluftkanal 21 und damit in das Saugrohr 23 Falschluft über das Sieb 53 angesaugt wird. Der Außenluftkanal 22 hat hierbei die Funktion einer Sperre gegen das Eindringen dieser Falschluft.

Nach dem Einregeln kann am Anzeige- und Bedienungspult 5 die im Heißfilmsensor 27 durchströmende Luftmenge sowie der Soll- und Meßdifferenzdruck als Maß für die Luftdurchlässigkeit des eingespannten Siebes 53 abgelesen werden. Evtl. Differenzdruckänderungen im Saugrohr 23 werden über das Feinregelventil 33 sofort korrigiert, während die Regeleinrichtung 40 dafür sorgt, daß keine Druckdifferenz zwischen Innenluftkanal 21 und Außenluftkanal 22 entsteht.

**Patentansprüche**

1. Prüfeinrichtung zur Messung der Luftdurchlässigkeit von textilen Materialbahnen, insbesondere von Papiermaschinenfilzen und -sieben, mit einer Meßgabel (2) mit im wesentlichen parallel und im Abstand zueinander verlaufenden Schenkeln (7, 8), an deren freien Enden je ein Rohrstutzen (12, 13) angebracht ist, von denen einer mittels einer Einspanneinrichtung zum Einspannen der Materialbahn zwischen den Rohrstutzen gegen den anderen bewegbar ist und von denen einer über einen Luftkanal, insbesondere Saugkanal, mit einem durch einen Motor angetriebenen Lüfter (24) bzw. Pumpe verbunden ist, wobei in dem Luftkanal zumindest eine Steuereinrichtung zur Einstellung des Volumenstroms, zumindest ein Unterdruckmesser — vorzugsweise im Bereich des angeschlossenen Rohrstutzens — sowie zumindest ein Luftdurchfluß- oder Luftvolumenmesser (27) angeordnet sind, dadurch gekennzeichnet, daß die Meßgabel (2) starre, von einem Rahmen, insbesondere einem Gehäuseteil (6), für den Lüfter (24) und dessen Motor ausgehende Schenkel (7, 8) aufweist, wobei einer der Rohrstutzen (12) im wesentlichen koaxial zu dem anderen Rohrstutzen (13) relativ zu dem betreffenden Schenkel (7) verschiebbar ist und der andere Rohrstutzen (13) mit dem als Luftrohr (23) ausgebildeten Luftkanal verbunden ist, das in dem betreffenden Schenkel (8) zum großen Teil gerade verläuft.

2. Prüfeinrichtung nach Anspruch 1, dadurch gekennzeichnet, daß der mit dem Luftrohr (23) verbundene Rohrstutzen als Doppelrohrstutzen (12, 13) mit jeweils koaxial zueinander angeordneten Innen- und Außenrohrteilen (17, 18 bzw. 19, 20) ausgebildet ist, daß das Luftrohr (23) an das Innenrohrteil (19) des betreffenden Doppelrohrstutzens (13) angeschlossen und das Außenrohrteil (20) an ein in dem betreffenden Schenkel verlaufendes, zwischen Luftdurchfluß- bzw. Luftvolumenmesser (27) und Sauglüfter (26) in das Luftrohr (25) mündendes Parallelrohr (28) verbunden ist, in dem ein Steuerventil (29) zur Einstellung des Unterdrucks angeordnet ist, und das Außenrohrteil (20) und Innenrohrteil (19) des unbewegbaren Doppelrohrstutzens (13) an einen zweiten Differenzdruckmesser (40) angeschlossen sind.

3. Prüfeinrichtung nach Anspruch 2, dadurch gekennzeichnet, daß beide Rohrstutzen als Doppelrohrstutzen (12, 13) ausgebildet sind, wobei die gegenüberliegenden Ränder von Innen- und Außenrohrteilen (17, 18 bzw. 19, 20) jeweils deckungsgleich sind.

4. Prüfeinrichtung nach Anspruch 2 oder 3, dadurch gekennzeichnet, daß das Steuerventil (29) mit einem Steuermotor verbunden ist, wobei der Steuermotor an einem mit dem zweiten Differenzdruckmesser (40) in Verbindung stehenden Unterdruckregler angeschlossen ist.

5. Prüfeinrichtung nach einem der Ansprüche 1 bis 4, dadurch gekennzeichnet, daß für die Bewegung des Rohrstutzens (12) ein Motor (16) zur Erzeugung eines definierten Anpressdrucks vorgesehen ist.

6. Prüfeinrichtung nach einem der Ansprüche 1 bis 5, dadurch gekennzeichnet, daß in alle einander zugewandten Stirnflächen der Rohrstutzen (12, 13) jeweils zumindest ein, vorzugsweise mehrere, konzentrische Rundschnurdichtringe (54, 55) mit quadratischem oder rundem Querschnitt eingesetzt sind.

7. Prüfeinrichtung nach einem der Ansprüche 1 bis 6, dadurch gekennzeichnet, daß der Luftdurchfluß- bzw. Luftvolumenmesser (27) im lüfterseitigen Endbereich des geraden Teils des Luftrohrs (23) angeordnet ist.

8. Prüfeinrichtung nach einem der Ansprüche 1 bis 7, dadurch gekennzeichnet, daß der erste Differenzdruckmesser (35) zumindest je eine Druckentnahme (38, 39) in den Innenrohrteilen (17, 19) der beiden Doppelrohrstutzen (12, 13) aufweist.

9. Prüfeinrichtung nach Anspruch 8, dadurch gekennzeichnet, daß die Rohrstutzen (12, 13) bzw. deren Innenrohrteil(en) (17, 19) über den Umfang verteilt mehrere — zumindest drei — Druckentnahmen (57, 59) aufweisen, die über je einen Ringkanal (56, 58) ggf. in dem bzw. den Innenrohrteil(en) (17, 19) verbunden sind.

10. Prüfeinrichtung nach einem der Ansprüche 1 bis 9, dadurch gekennzeichnet, daß an das Luftrohr (23) zwischen Luftdurchfluß- bzw. Luftvolumenmesser (27) und Lüfter (24) bzw. Pumpe zumindest eine Bypass-Leitung (30) mit darin angeordneten Regelventilen (32) angeschlossen ist.

11. Prüfeinrichtung nach Anspruch 10, dadurch gekennzeichnet, daß eine große Bypass-Leitung (30) mit einem Grobregelventil (32) und eine kleinere Bypass-Leitung (31) mit einem Feinregelventil (33) vorgesehen ist.

12. Prüfeinrichtung nach Anspruch 11, dadurch gekennzeichnet, daß das Grob- und Feinregelventil (32, 33) jeweils mit einem Verstellmotor versehen sind, wobei der Verstellmotor des Grobregelventils (32) an einem Dreipunktregler mit einstellbarem Totbereich angeschlossen ist.

13. Prüfeinrichtung nach einem der Ansprüche 8 oder 9 und 10 bis 12, dadurch gekennzeichnet, daß der erste Differenzdruckmesser (35) und das bzw. die Regelventile (32, 33) in einem Regelkreis zur Einstellung und zum Konstanthalten des Differenzdrucks angeordnet sind.

**Revendications**

1. Dispositif de contrôle pour mesurer la perméabilité à l'air de nappes de tissus textiles, notamment de feutres de machines à papier et de toiles de machines à papier, comprenant une fourchette de mesure (2) ayant des branches (7, 8) qui s'étendent sensiblement parallèlement et à distance l'une de l'autre, et aux extrémités libres desquelles sont montées respectivement des embouts tubulaires (12, 13), dont l'un peut être déplacé par rapport à l'autre au moyen d'un dispositif de blocage destiné à bloquer la nappe de tissu entre les embouts tubulaires, et dont l'autre communique, par un conduit pour de l'air, notamment par un conduit d'aspiration, avec un ventilateur (24) ou avec une pompe entraîné par un moteur, avec, dans le conduit pour de l'air, au moins un dispositif de commande pour régler le courant en volume, au moins un déprimomètre -de préférence dans la région du raccordement de l'embout tubulaire- ainsi qu'au moins un dispositif de mesure du débit ou du volume d'air (27), caractérisé en ce que la fourchette de mesure (2) comporte des branches (7, 8) rigides, issues d'un bâti, notamment d'une partie formant enveloppe (6), pour le ventilateur (24) et son moteur, l'un des embouts tubulaires (12) pouvant être déplacé par rapport à la branche (7) le concernant sensiblement coaxialement à l'autre embout tubulaire (13) et ce dernier (13) communiquant avec le conduit pour l'air qui est constitué sous la forme d'un tuyau pour l'air (23) et qui, pour la plus grande partie, s'étend de façon rectiligne dans la branche (8) la concernant.

2. Dispositif de contrôle suivant la revendication 1, caractérisé en ce que l'embout tubulaire communiquant avec le tube pour l'air (23) est agencé en embout tubulaire double (12, 13) ayant des parties tubulaires intérieure et extérieure (17, 18 et 19, 20) disposées coaxialement l'une à l'autre, en ce que le tuyau pour l'air (23) est raccordé à la partie tubulaire intérieure (19) de l'embout tubulaire double (13) le concernant et en ce que la partie tubulaire extérieure (20) communique avec un tuyau parallèle (28) qui s'étend dans la branche le concernant, qui débouche dans le tuyau pour l'air (25) entre le dispositif de mesure du débit ou du volume d'air (27) et le ventilateur d'aspiration (26) et dans lequel est montée une vanne de commande (29) pour régler la dépression, et en ce que la partie tubulaire extérieure (20) et la partie tubulaire intérieure (19) de l'embout tubulaire double (13) fixe sont raccordées à un second manomètre différentiel (40).

3. Dispositif de contrôle suivant la revendication 2, caractérisé en ce que les deux embouts tubulaires sont agencés en embout tubulaire double (12, 13) les bords opposés des parties tubulaires intérieure et extérieure (17, 18 et 19, 20) étant respectivement en coïncidence.

4. Dispositif de contrôle suivant la revendication 2 ou 3, caractérisé en ce que la vanne de commande (29) est reliée à un moteur de commande, le moteur de commande étant relié à un dispositif de réglage de dépression communiquant avec le second manomètre différentiel.

5. Dispositif de contrôle suivant l'une des revendications 1 à 4, caractérisé en ce que pour le déplacement de l'embout tubulaire (12), il est prévu un moteur (16) produisant une pression d'application définie.

6. Dispositif de contrôle suivant l'une des revendications 1 à 5, caractérisé en ce que, dans toutes les surfaces frontales des embouts tubulaires (12, 13), qui sont tournées l'une vers l'autre, sont introduits respectivement au moins un, et de préférence plusieurs joints annulaires (54, 55) concentriques de section transversale carrée ou ronde.

7. Dispositif de contrôle suivant l'une des revendications 1 à 6, caractérisé en ce que le dispositif de mesure du débit ou du volume d'air (27) est disposé dans la région d'extrémité de la partie rectiligne du tuyau pour l'air (23), qui se trouve du côté du ventilateur.

8. Dispositif de contrôle suivant l'une des revendications 1 à 7, caractérisé en ce que le premier manomètre différentiel (35) comporte au moins une prise de pression (38, 39) dans chacune des parties tubulaires intérieures (17, 19) des deux embouts tubulaires doubles (12, 13).

9. Dispositif de contrôle suivant la revendication 8, caractérisé en ce que les embouts tubulaires (12, 13) et leur partie tubulaire intérieure (17, 19) présentent, d'une manière répartie sur le pourtour, plusieurs -au moins trois- prises de pression (57, 59) qui communiquent chacune par un canal annulaire (56, 58), le cas échéant, avec la ou les parties tubulaires intérieures (17, 19).

10. Dispositif de contrôle suivant l'une des revendications 1 à 9, caractérisé en ce que, au tuyau pour l'air (23), est raccordé, entre le dispositif de mesure du débit ou du volume d'air (27) et le ventilateur (24) ou la pompe, au moins un conduit de dérivation (30) dans lequel sont montées des vannes de réglage (32).

11. Dispositif de contrôle suivant la revendication 10, caractérisé en ce qu'il est prévu un grand conduit de dérivation (30) avec une vanne de réglage grossier (32) et un conduit de dérivation plus petit (31) avec une vanne de réglage fin (33).

12. Dispositif de contrôle suivant la revendication 11, caractérisé en ce que la vanne de réglage grossier (32) et la vanne de réglage fin (33) sont munies chacune d'un servomoteur, le servomoteur de la vanne de réglage grossier (32) étant connecté à un dispositif de réglage par plus ou par moins à plage morte variable.

13. Dispositif de contrôle suivant l'une des revendications 8 ou 9, et 10 à 12, caractérisé en ce que le premier manomètre différentiel (35) et la ou les vannes de réglage (32, 33) sont montés dans un circuit de réglage destiné à régler et à maintenir constante la pression différentielle.

## Claims

1. Testing apparatus for measuring the permeability to air of textile material webs, especially papermaking machine felts and wires, with a measuring fork (2) having arms (7, 8) which are situated substantially parallel and in spaced-apart relationship relatively to one another and at each of the free ends of which a stub pipe (12, 13) is arranged, one of the said pipes being movable towards the other by means of a clamping device for clamping the web of material between the stub pipes, and one of said pipes being connected via an air duct, especially a suction duct, to a pump or fan (24) driven by a motor, there being arranged in the air duct at least one control device for adjusting the volume flow, at least one negative-pressure meter—preferably in the region of the connected stub pipe—and also at least one air-throughflow or air-volume meter (27), characterised in that the measuring fork (2) comprises rigid arms (7, 8) emanating from a frame, especially a housing part (6), for the fan (24) and the fan motor, one of the stub pipes (12) being displaceable substantially coaxially with the other stub pipe (13) relatively to the relevant arm (7) and the other stub pipe (13) being connected to the air duct which is constructed as an air pipe (23) and which follows a straight course for the greater part in the relevant arm (8).

2. Testing apparatus according to claim 1, characterised in that the stub pipe connected to the air pipe (23) is constructed as a double stub pipe (12, 13) with inner and outer pipe parts (17, 18 and 19, 20 respectively) which are situated coaxially with each other in each case, that the air pipe (23) is connected to the inner pipe part (19) of the relevant double stub pipe (13) and the outer pipe part (20) is connected to a parallel pipe (28) which is arranged in the relevant arm and which opens into the air pipe (25) between the air-throughflow or air-volume meter (27) and the suction fan (26) and in which a control valve (29) for adjustment of the negative pressure is arranged, and the outer pipe part (20) and inner pipe part (19) of the non-movable double stub pipe (13) are connected to a second differential pressure meter (40).

3. Testing apparatus according to claim 2, characterised in that both stub pipes are constructed as double stub pipes (12, 13), the opposite edges of inner and outer pipe parts (17, 18 and 19, 20 respectively) being in exact register in each case.

4. Testing apparatus according to claim 2 or 3, characterised in that the control valve (29) is connected to a control motor, the control motor being connected to a negative-pressure controller which is connected to the second differential pressure meter (40).

5. Testing apparatus according to one of claims 1 to 4, characterised in that a motor (16) for producing a specific application pressure is provided for the movement of the stub pipe (12).

6. Testing apparatus according to one of claims 1 to 5, characterised in that at least one in each case, preferably a plurality of, concentric toroidal sealing rings (54, 55) with a square or round cross-section is/are inserted in all the mutually facing end faces of the stub pipes (12, 13).

7. Testing apparatus according to one of claims 1 to 6, characterised in that the air-throughflow or air-volume meter (27) is arranged in that end region of the straight portion of the air pipe (27) which is at the fan side.

8. Testing apparatus according to one of claims 1 to 7, characterised in that the first differential pressure meter (35) has at least one pressure pickup (38, 39) in each of the inner pipe parts (17, 19) of the two double stub pipes (12, 13).

9. Testing apparatus according to claim 8, characterised in that the stub pipes (12, 13), or their inner pipe part/parts (17, 19), comprise, distributed circumferentially, a plurality of at least three pressure pickups (57, 59) connected in each case by an annular duct (56, 58) if appropriate in the inner pipe part or parts (17, 19).

10. Testing apparatus according to one of claims 1 to 9, characterised in that at least one bypass conduit (30) with regulating valves (32) arranged therein is connected to the air pipe (23) between the air-throughflow or air-volume meter (27) and the fan (24) or pump.

11. Testing apparatus according to claim 10, characterised in that a large bypass conduit (30) is provided with a coarse regulating valve (32) and a relatively small bypass conduit (31) is provided with a fine-regulating valve (33).

12. Testing apparatus according to claim 11, characterised in that the coarse-regulating and fine-regulating valves (32, 33) are each provided with an adjusting motor, the adjusting motor of the coarse-regulating valve (32) being connected to a three-point controller with an adjustable dead range.

13. Testing apparatus according to one of claims 8 or 9 and 10 to 12, characterised in that the first differential pressure meter (35) and the regulating valve or valves (32, 33) are arranged in a control circuit for adjusting and keeping constant the differential pressure.

Fig.1

EP 0 096 311 B1

Fig.3

Fig.2

Schnitt A-B

Fig. 4